# EUROPÄISCHE PATENTSCHRIFT

(11) **EP 0 834 327 B1**
(45) Veröffentlichungstag und Bekanntmachung des Hinweises auf die Patenterteilung: **27.10.2004**
(21) Anmeldenummer: 97113344.2
(22) Anmeldetag: 01.08.1997
(51) Int. Cl.: A61M 1/16, A61L 2/18

(54) **Dialysemaschine und Verfahren zu deren Desinfektion**
Dialysis machine and method for its desinfection
Machine pour la dialyse et procédé pour sa désinfection

(30) Priorität: 02.10.1996 DE 19640840
(43) Veröffentlichungstag der Anmeldung: 08.04.1998
(73) Patentinhaber: Fresenius Medical Care Deutschland GmbH, 61350 Bad Homburg v.d.H. (DE)
(72) Erfinder: Spickermann, Reiner, 97535 Wasserlosen/Burghausen (DE)
(74) Vertreter: Laufhütte, Dieter, Dr.-Ing.

(56) Entgegenhaltungen:
- WO-A-93/09821
- DE-A- 2 428 256
- US-A- 5 484 397
- US-A- 5 585 003

## Beschreibung

Die Erfindung betrifft eine Dialysemaschine und ein Verfahren zu deren Desinfektion nach dem Oberbegriff der Ansprüche 1 und 10.

Dialysemaschinen müssen vor jeder Benutzung desinfiziert bzw. in einen möglichst sterilen Zustand gebracht werden.

Herkömmlicherweise werden den Dialysemaschinen die benötigten Desinfektionsmittel in verwendungsfertigem Zustand von außen zugeführt.

Dies führt zu hohen Transport- und Verpackungskosten. Im übrigen entsteht so eine Abhängigkeit von der tatsächlichen Verfügbarkeit der benötigten Desinfektionsmittel. Darüber hinaus ist das Handling solcher Stoffe nur unter Beachtung besonderer Vorsichtsmaßnahmen möglich, da es sich in der Regel um aggressive, ätzende Substanzen handelt. Das für die Anlieferung dieser Substanzen verwendete Verpackungsmaterial muss ferner entsorgt werden. Als besonders kostenintensiv erweisen sich jedoch in der Praxis die Transportprobleme.

Der weltweit am häufigsten zur Desinfektion von Dialysegeräten eingesetzte Wirkstoff ist NaOCl (Bleach). Bei diesem hochwirksamen Desinfektions- und Bleichmittel ist jedoch die Umweltbelastung besonders groß. Andererseits weist NaOCl jedoch Vorteile bezüglich der einfachen Handhabung und der effektiven Reinigung/Desinfektion auf. Ferner ist NaOCl in zahlreichen Staaten oftmals das einzig verfügbare Desinfektionsmittel.

Es finden aber auch andere Desinfektionsmittel, wie z.B. Peressigsäure, Wasserstoffperoxid, Zitronensäure oder Ozon, breite Verwendung.

Aus der US 5,484,397 ist eine Dialysevorrichtung bekannt, bei der Desinfektionsmittel vor ihrer Verwendung in einer Kammer aufgenommen sind und bei Bedarf in Wasser gelöst werden. Darüber hinaus weist die Dialysevorrichtung einen Ozongenerator auf, mittels dessen Ozon in dem Dialysegerät hergestellt werden kann und sodann für Desinfektionszwecke zur Verfügung steht.

Aufgabe der Erfindung ist es, eine Dialysemaschine zur Verfügung zu stellen, mittels derer gewährleistet werden kann, dass umweltbelastende Desinfektionsmittel nach ihrer Benutzung nicht in das Abwasser bzw. die Außenwelt gelangen.

Diese Aufgabe wird durch eine Dialysemaschine gemäß Patentanspruch 1 sowie durch ein Verfahren gemäß Patentanspruch 10 gelöst. Vorteilhafte Ausgestaltungen der Erfindung sind Gegenstand der Unteransprüche.

Mit der erfindungsgemäßen Dialysemaschine bzw. dem erfindungsgemäßen Verfahren ist es möglich, Dialysegeräte weitgehend unabhängig von der Verfügbarkeit fertiger Desinfektionsmittel zu betreiben, wobei es zu beachtlichen Ersparnissen von Transport- und Verpackungskosten kommt. Eine Entsorgung des üblicherweise benötigten Verpackungsmaterials entfällt ebenso wie weite Gefahrentransporte mit Chemikalien. Für den Bediener ergeben sich zudem weitaus größere Handlingsvorteile.

Ferner ist die Betreibersicherheit insofern erhöht, als ein Umgang mit aggressiven, ätzenden Substanzen weitgehend vermieden wird.

Gemäß der vorliegenden Erfindung weist die Dialysemaschine Mittel zur Zersetzung des onlinegenerierten Desinfektionsmittels in umweltverträgliche Zersetzungsprodukte auf. Hierdurch ist gewährleistet, dass umweltbelastende Desinfektionsmittel nach ihrer Benutzung nicht in das Abwasser bzw. die Außenwelt gelangen.

Gemäß einer vorteilhaften Ausgestaltung weist die Dialysemaschine eine Leitungseinrichtung auf, über welche bei der Zersetzung des wenigstens einen Desinfektionsmittels entstehende Zersetzungsprodukte, welche zur Online-Generierung von Desinfektionsmittel wiederverwendbar sind, zu den Mitteln zur Online-Generierung von Desinfektionsmittel zurückführbar sind. Hierdurch ist ein geschlossener Recycling-Kreislauf zur Desinfektionsmittel-Herstellung geschaffen.

Gemäß der vorliegenden Erfindung weist die Dialysemaschine einen NaOCl-Generator auf, welcher Natriumhypochlorit (NaOCl) durch Elektrolyse von NaCl erzeugt. Besonders bevorzugt ist hierzu die Verwendung von im Dialysatkonzentrat vorhandenen NaCl.

Die erfindungsgemäße Dialysemaschine weist nach einer vorteilhaften Ausgestaltung eine Bleidioxidanode zur Herstellung von Ozon durch Elektrolyse von Sauerstoff auf.

Gemäß einer weiteren vorteilhaften Ausgestaltung ist ferner eine UV-Photooxidationseinrichtung vorgesehen, mittels der eine UV-Photooxidation von Wasser durchgeführt werden kann. Es ist auch möglich, ein Dialysekonzentrat oder auch ein verwendungsfertiges Dialysat einer UV-Photooxidation auszusetzen. Hierbei wird beispielsweise in Wasser gelöster Sauerstoff in Ozon umgewandelt. Dieses Ozon wirkt keimtötend und kann daher zur Herstellung von keimfreiem Wasser verwendet werden.

Das so erzeugte keimfreie Wasser kann der Desinfektionsflüssigkeit beigegeben werden. In der Regel ist davon auszugehen, dass das durch UV-Photooxidation erzeugte Ozon zusammen mit weiterem Ozon verwendet wird.

Während der Desinfektion ist jedoch das keimfreie Wasser nicht notwendig. Die Verwendung ist vor allem in dem Nachspülvorgang zu sehen, jedoch auch zur Herstellung von Substituat für Hämofiltration und Hämodiafiltration aus ultrareinem Wasser, da dieses direkt dem Patienten infundiert wird. Des weiteren kann Dialysat ultrarein erzeugt werden oder beispielsweise über den Dialysator der Blutkreislauf vorgefüllt werden.

Das durch UV-Photooxidation erzeugte keimfreie Wasser ist auch als Spülwasser verwendbar, mittels dessen nach Beendigung des Desinfektionsvorgangs Desinfektionsmittel-Rückstände aus der Dialysemaschine entfernt werden können. Durch die Erzeugung von Ozon im vorgereinigten Wasser wird gewährleistet, dass während des Freispülvorgangs keine erneute Kontamination der Dialysemaschine durch das Spülwasser stattfinden kann.

Es ist möglich, mittels einer reversen Osmose erzeugtes Wasser (RO-Wasser) in ultrareines Wasser umzuwandeln. Diesem ultrareinen Wasser kann dann beispielsweise jedes gewünschte Desinfektionsmittel zugegeben werden, womit eine optimale Desinfektion der Dialysemaschine gewährleistet ist. Das so erzeugte ultrareine Wasser kann, wie erwähnt, anschließend als Spülwasser verwendet werden. Ferner ist es bei der anschließenden Dialyse zur Verdünnung eines Dialyselösung-Konzentrats verwendbar, womit ultrareines Dialysat bereitet werden kann.

Gemäß einer vorteilhaften Ausgestaltung weist die Dialysemaschine einen Katalysator zur katalytischen Zersetzung von NaOCl in NaCl und Sauerstoff auf.

Das hierdurch entstehende NaCl kann entweder dem Abwasser zugeleitet werden, womit die Belastung des Abwassers stark reduziert ist, oder, gemäß einer weiteren Ausführungsform, dem NaOCl-Generator wieder zugeführt werden.

Gemäß einer weiteren vorteilhaften Ausgestaltung weist die Dialysemaschine ferner eine Vorrichtung zur Bestimmung des Desinfektionsmittelgehaltes in der verwendeten Desinfektionsflüssigkeit bzw. in einer anschließend verwendeten Spülflüssigkeit auf.

Bei dieser Vorrichtung zur Bestimmung des Desinfektionsmittelgehaltes handelt es sich bevorzugt um eine Redox-Elektrode. Dadurch kann zum einen sichergestellt werden, daß Desinfektionsmittel tatsächlich vorhanden ist, zum anderen nach dem Freispülvorgang getestet werden, ob sich noch Desinfektionsmittel im Dialysatkreislauf befindet. Eine solche Redox-Elektrode kann ohne weiteres in eine Dialysemaschine integriert werden.

Die Bestimmung des Desinfektionsmittelgehaltes über das Redoxpotential der Desinfektionsflüssigkeit, der Spülflüssigkeit bzw. der bei der eigentlichen Dialyse verwendeten Dialyselösung stellt ein weiteres einfaches und zuverlässiges Verfahren dar. Ein von der Redox-Elektrode abgenommener Meßwert kann vorteilhaft als Steuergröße für die Steuerung des Spül- und Katalysevorgangs verwendet werden.

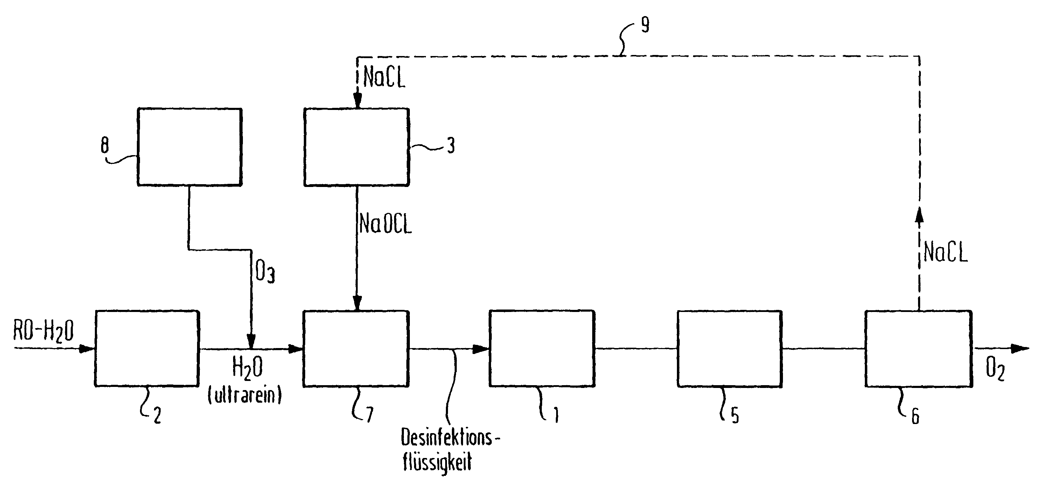

Ein Ausführungsbeispiel der Erfindung wird nachstehend anhand der Zeichnung näher erläutert, in deren einziger Figur schematisch die einzelnen Bestandteile einer erfindungsgemäßen Dialysemaschine bzw. die entsprechenden Verfahrensschritte dargestellt sind.

Es sei darauf hingewiesen, daß erfindungsgemäß die dargestellten Bestandteile durch entsprechende Mittel innerhalb des Dialysegerätes ausgeführt sein können. Es ist jedoch gleichfalls möglich, ein herkömmliches Dialysegerät extern mit Zusatzmodulen zu verbinden, welche mit derartigen Mitteln ausgestattet sind. Diese Ausführungsform ist jedoch nicht erfindungsgemäß.

Blut von einem Patienten wird einer zentralen Dialyseeinheit 1 zugeführt und in dieser Dialyseeinheit 1 gereinigt bzw. dialysiert. Dann wird das Blut dem Patienten wieder zugeführt. Die vorliegende Erfindung behandelt eine Desinfektion der Leitungen bzw. Abschnitte der Dialyseeinheit, durch die Dialysat fließt. Die Blutseite besteht in der Regel aus Wegwerfteilen (Disposable). Zum Reinigen und Desinfizieren der Dialysatseite, wird der Dialysator (Hohlfaserfilter) zusammen mit dem Disposable entfernt und verworfen, die Anschlüsse zu dem Dialysator werden kurzgeschlossen. In Fällen der Wiederbenutzung des Dialysators (Reuse) kann er auch im Kreislauf verbleiben und mitdesinfiziert werden. Dazu ist aber ein membranverträgliches Desinfektionsmittel nötig. Die Trennung von der Blutseite erfolgt über die semipermeable Membran des Dialysators selbst.

RO-Wasser wird einer UV-Photooxidationskammer 2 zugeführt. Das Wasser wird dort einer UV-Bestrahlung, beispielsweise der UV-Strahlung einer Quecksilber-Niederdrucklampe, ausgesetzt. UV-Licht im Spektralbereich von 200 - 300 Nanometern tötet Mikroorganismen, indem es die DNA der Zellen zerstört. Quecksilber-Niederdrucklampen erzeugen die größte Energie bei einer Wellenlänge von 254 Nanometern. Dieser Wert liegt ganz in der Nähe der optimalen Wellenlänge zur Entkeimung, welche 260 Nanometer beträgt.

Speziell entwickelte Lampen, für die nur hochreines Quarz verwendet wird, ermöglichen ferner die Abstrahlung einer Wellenlänge bei 185 nm. Der kombinierte Effekt, der durch UV-Licht mit den Wellenlängen 185 nm und 254 nm entsteht, bewirkt die Photooxidation von gelösten organischen Verbindungen.

Die Emission von UV-Licht 185 nm katalysiert die Reaktion von im Wasser gelösten Sauerstoff zu Ozon. Das so erzeugte Ozon bewirkt bei seinem Zerfall eine Abtötung der im Wasser anwesenden Keime.

Insgesamt läßt sich durch die UV-Photooxidation des RO-Wassers ultrareines Wasser herstellen. Bei Bedarf kann diesem ultrareinen Wasser weiteres Ozon zur Desinfektion zugegeben werden, wie dies schematisch in der Zeichnung dargestellt ist. In der Regel wird jedoch das ultrareine Wasser zum Nachspülen verwendet, um Desinfektionsmittel wieder zu entfernen. Desweiteren wird es zur Verdünnung von Dialysekonzentrat eingesetzt.

Das zugegebene Ozon kann mittels Wasserelektrolyse mit Bleidioxid-Anoden 8 hergestellt werden oder mit Hilfe von Siemens-Verfahren. Die Benutzung dieses Verfahrens der elektrolytischen Oxidation mit Bleidioxid-Anoden bietet insofern Vorteile gegenüber anderen Erzeugungsverfahren, als keine Gasbildung erfolgt und hohe Konzentrationen erzielbar sind. Selbstverständlich sind auch andere Verfahren zur Erzeugung von Ozon denkbar. Ferner ist die Zuführung bereits fertig hergestellten Ozons möglich.

In einem Generator 3 erfolgt die elektrolytische Herstellung von NaOCl. Beim bevorzugten Herstellungsverfahren wird ca. 10%ige Natriumchloridlösung derart elektrolysiert, daß das am Pluspol freiwerdende Chlor und das am Minuspol entstehende NaOH miteinander reagieren nach der Gleichung Cl2 + 2NaOH = NaOCl + NaCl + H20.

Das hierdurch enstandene NaOCl wird dem (ultrareinen) Wasser im gewünschten Verhältnis zugeführt, benötigt jedoch kein ultrareines Wasser, um die Desinfektionswirkung auszuüben.

Das so erzeugte Desinfektionsmittel wird über eine Zuführeinrichtung 7 der zentralen Dialyseeinheit 1 zugeführt.

Ozon weist insofern Vorteile auf, als es zu einer weniger starken Beeinträchtigung einer Dialysemembran führt, falls der Dialysator in der Maschine verbleibt, oder ein sogenannter Diasafe- oder Online-Filter in der Dialysemaschine ist.

Ferner weist Ozon bezüglich der Abwasserbelastung den Vorteil auf, daß es relativ instabil ist und sich nach einiger Zeit zersetzt.

Es ist ebenfalls denkbar, für besondere Anwendungsfälle die Desinfektion eines medizinischen Gerätes ausschließlich mittels Ozon zu gewährleisten.

Ozon ist auch vorteilhaft bei der keimfreien Konservierung von Wasser über mehrere Tage verwendbar. Insbesondere eine Heimdialysemaschine wird nur alle zwei bis drei Tage benutzt. Ein ausreichender Ozongehalt des im Gerät verbleibenden Wassers verhindert Keimwachstum bis zur nächsten Benutzung.

Mit dem hergestellten Ozon bzw. dem hergestellten NaOCl wird die Dialysemaschine desinfiziert. Sie muß jedoch vor dem eigentlichen Dialysevorgang mit einer Spülflüssigkeit nachgespült werden, um Reste des Desinfektionsmittels zu beseitigen. Um die Maschine durch Keime im Spülwasser nicht erneut zu kontaminieren, wird als Spülwasser sowohl RO-Wasser als auch mittels einer Photooxidation keimfrei gemachtes Wasser verwendet. Die Kombination wird als besonders bevorzugt angesehen. Auch bei der anschließenden Dialyse kann dieses keimfreie Wasser genutzt werden, um ein Dialyse-Lösungskonzentrat in gewünschter Weise zu verdünnen.

Zur Überwachung einer einwandfreien Desinfektion bzw. Nachspülung ist eine Redox-Elektrode 5 vorgesehen, mittels derer das Redox-Potential von Flüssigkeiten bestimmbar ist. Durch die Bestimmung des Redox-Potentials ist somit der Desinfektionsmittelgehalt bestimmbar. Die Redox-Elektrode dient zum Nachweis der Anwesenheit einer ausreichenden Menge Desinfektionsmittel während des Desinfektionsvorgangs, um so einen korrekten Ablauf des Desinfektionsprogramms sicherzustellen. Ferner dient sie zum Nachweis des korrekten Freispülens der Dialysemaschine, bevor diese zur erneuten Dialyse an Patienten angeschlossen wird.

Die Redox-Elektrode dient in erster Linie zur Detektion von Desinfektionsmittel, falls nicht vollständig freigespült wurde.

Die mittels der Redox-Elektrode bestimmten Messwerte sind zusätzlich zur Anwesenheitsbestimmung als Steuergröße für die Steuerung des Desinfektions-, Spül- und Katalysevorgangs verwendbar.

Eine solche Redox-Elektrode lässt sich in ein Dialysegerät einbauen. Der zur Zeit noch manuell durchzuführende Nachweis des Freispülens, beispielsweise mittels Teststreifen (KJ-Stärkepapier, Peroxidtest, etc.) kann entfallen.

Durch das Vorsehen einer Redox-Elektrode ist eine einfache Kontrolle des Desinfektionsprogrammes möglich und eine zusätzliche Schutzfunktion während der Dialyse gegeben.

Schließlich wird die gebrauchte Desinfektionsflüssigkeit einer Katalysatorvorrichtung 6 zugeführt, welche die katalytische Zersetzung des NaOCl in die Bestandteile NaCl und O2 bewirkt.

Diese Katalysatorvorrichtung 6 ist im Dialysegerät integriert.

Das bei der katalytischen Reaktion erzeugte NaCl kann dann über eine Leitungseinrichtung 9 dem NaOCl-Generator 3 zugeführt werden. Somit ist ein geschlossener Kreislauf zur Herstellung und katalytischen Zersetzung von NaOCl realisiert. Die Ausgangsstoffe NaCl und H2O reagieren zu NaOCl, welches nach seiner katalytischen Zersetzung in NaCl und O2 zerfällt.

Von diesen Ausgangsprodukten gehen keine Gefahren aus. Sowohl eine Verätzungsgefahr für den Bediener des Gerätes, als auch eine Gefahr für die Umwelt kann ausgeschlossen werden.

Die verschiedenen Vorrichtungen können innerhalb eines Dialysegerätes angeordnet werden. Es ist somit ein integriertes Dialysegerät zur Verfügung gestellt, welches eine wesentliche Bedienungsvereinfachung gegenüber herkömmlichen Geräten aufweist.

Wie erwähnt, wird das erfindungsgemäße Verfahren integriert in einem solchen Dialysegerät durchgeführt . Es ist jedoch gleichfalls möglich, wenigstens einen der Verfahrensschritte UV-Photooxidation, Ozon-Erzeugung, Bestimmung des Desinfektionsmittelgehaltes mittels Redox-Elektrode extern, d.h. über eine zentrale Versorgungseinheit, durchzuführen.

## Patentansprüche

1. Dialysemaschine mit Mitteln zur Online-Generierung wenigstens eines zur Desinfektion der Dialysemaschine verwendbaren Desinfektionsmittels,
**dadurch gekennzeichnet,**
**dass** die Mittel einen Generator (3) zur Herstellung von NaOCl durch Elektrolyse von NaCl umfassen und dass die Dialysemaschine ferner Mittel (6) zur Zersetzung des onlinegenerierten Desinfektionsmittels in umweltverträgliche Zersetzungsprodukte aufweist.

2. Dialysemaschine nach Anspruch 1, **dadurch gekennzeichnet, dass** sie Mittel zur Herstellung von Ozon, insbesondere eine Bleidioxidelektrode oder eine Vorrichtung zur Durchführung eines Siemens-Verfahrens, aufweist.

3. Dialysemaschine nach einem der vorstehenden Ansprüche, **dadurch gekennzeichnet, dass** sie eine UV-Photooxidationseinrichtung aufweist.

4. Dialysemaschine nach einem der vorstehenden Ansprüche, **dadurch gekennzeichnet, dass** sie einen Katalysator (6) zur katalytischen Zersetzung von Desinfektionsmitteln aufweist.

5. Dialysemaschine nach Anspruch 4, **dadurch gekennzeichnet, dass** sie einen Katalysator zur katalytischen Zersetzung von NaOCl aufweist.

6. Dialysemaschine nach Anspruch 5, **dadurch gekennzeichnet, dass** sie eine Leitungseinrichtung aufweist, über welche das bei der katalytischen Zersetzung erzeugte NaCl dem NaOCl-Generator (3) wiederzuführbar ist.

7. Dialysemaschine nach einem der vorstehenden Ansprüche, **dadurch gekennzeichnet, dass** sie eine Leitungseinrichtung aufweist, über welche bei der Zersetzung des wenigstens einen Desinfektionsmittels entstehende Zersetzungsprodukte, welche zur Online-Generierung von Desinfektionsmittel wiederverwendbar sind, zu den Mitteln zur Online-Generierung von Desinfektionsmittel zurückführbar sind.

8. Dialysemaschine nach einem der vorstehenden Ansprüche, **dadurch gekennzeichnet, dass** sie Mittel (5) zur Bestimmung des Desinfektionsmittelgehaltes bzw. der Anwesenheit von Desinfektionsmitteln einer bei der Desinfektion verwendeten Desinfektionsflüssigkeit, einer zum Ausspülen der Desinfektionsflüssigkeit verwendeten Spülflüssigkeit und/oder einer im Dialysatkreislauf verwendeten Dialysatflüssigkeit aufweist.

9. Dialysemaschine nach Anspruch 8, **dadurch gekennzeichnet, dass** sie eine Redox-Elektrode zur Bestimmung des Desinfektionsmittelgehaltes aufweist.

10. Verfahren zur Desinfektion einer Dialysemaschine, bei dem die keimfrei zu haltenden Bereiche der Dialysemaschine mit einem Desinfektionsmittel desinfiziert werden, wobei das Desinfektionsmittel online von Mitteln zur Generierung wenigstens eines Desinfektionsmittels erzeugt wird,
**dadurch gekennzeichnet,**
**dass** die Erzeugung des Desinfektionsmittels die Herstellung von NaOCl durch Elektrolyse von NaCl umfasst und dass in der Dialysemaschine eine Zersetzung des onlinegenerierten Desinfektionsmittels in umweltverträgliche Zersetzungsprodukte nach dem Desinfektionsvorgang erfolgt.

11. Verfahren nach Anspruch 10, **dadurch gekennzeichnet, dass** die keimfrei zu haltenden Bereiche der Dialysemaschine durch Zugabe von NaOCl als Desinfektionsflüssigkeit desinfiziert werden, und das hierbei verwendete NaOCl anschließend katalytisch in NaCl und weitere Reaktionsprodukte zersetzt wird.

12. Verfahren nach Anspruch 11, **dadurch gekennzeichnet, dass** das durch die katalytische Zersetzung erzeugte NaCl wenigstens teilweise zur Erzeugung von NaOCl wiederverwendet werden kann.

13. Verfahren nach Anspruch 10, **dadurch gekennzeichnet, dass** Ozon als Desinfektionsmittel verwendet wird.

14. Verfahren nach einem der Ansprüche 10-13, **dadurch gekennzeichnet, dass** das Vorhandensein von Desinfektionsmitteln festgestellt wird.

15. Verfahren nach einem der Ansprüche 10-14, **dadurch gekennzeichnet, dass** ultrareines Wasser durch Photooxidation hergestellt wird.

16. Verfahren nach Anspruch 15, **dadurch gekennzeichnet, dass** die Dialysemaschine nach erfolgter Desinfektion mit ultrareinem Wasser gespült wird.

## Claims

1. A dialysis machine having means for online generation of at least one disinfectant which can be used for disinfecting the dialysis machine,
**characterised in that**
the means include a generator (3) for producing NaOCl by electrolysis of NaCl and that the dialysis machine further has means (6) for decomposition of the online-generated disinfectant into environmentally compatible decomposition products.

2. A dialysis machine according to claim 1 **characterised in that** it has means for the production of ozone, in particular a lead oxide electrode or a device for carrying out a Siemens process.

3. A dialysis machine according to one of the preceding claims **characterised in that** it has a UV photo-oxidation device.

4. A dialysis machine according to one of the preceding claims **characterised in that** it has a catalyst (6) for catalytic decomposition of disinfectants.

5. A dialysis machine according to claim 4 **characterised in that** it has a catalyst for catalytic decomposition of NaOCl.

6. A dialysis machine according to claim 5 **characterised in that** it has a conduit device, by way of which the NaCl produced in the catalytic decomposition operation can be fed to the NaOCl generator (3) again.

7. A dialysis machine according to one of the preceding claims **characterised in that** it has a conduit device by way of which decomposition products which are produced in the decomposition of the at least one disinfectant and which can be re-used for online generation of disinfectant can be fed back to the means for online generation of disinfectant.

8. A dialysis machine according to one of the preceding claims **characterised in that** it has means (5) for determining the disinfectant content or the presence of disinfectants of a disinfecting fluid used in the disinfecting operation, a rinsing fluid used for rinsing out the disinfecting fluid and/or a dialysate fluid used in the dialysate circuit.

9. A dialysis machine according to claim 8 **characterised in that** it has a redox electrode for determining the disinfectant content.

10. A method of disinfecting a dialysis machine in which the regions of the dialysis machine which are to be kept germ-free are disinfected with a disinfectant, the disinfectant being produced online by means for generating at least one disinfectant,
**characterised in that**
production of the disinfectant includes the production of NaOCl by the electrolysis of NaCl and in the dialysis machine decomposition of the online-generated disinfectant into environmentally compatible decomposition products is effected after the disinfecting operation.

11. A method according to claim 10 **characterised in that** the regions of the dialysis machine which are to be kept germ-free are disinfected by the addition of NaOCl as disinfecting fluid and the NaOCl used **in that** case is then catalytically decomposed into NaCl and further reaction products.

12. A method according to claim 11 **characterised in that** the NaCl produced by the catalytic decomposition operation can be at least partially re-used for producing NaOCl.

13. A method according to claim 10 **characterised in that** ozone is used as the disinfectant.

14. A method according to one of claims 10 to 13 **characterised in that** the presence of disinfectants is detected.

15. A method according to one of claims 10 to 14 **characterised in that** ultra-pure water is produced by photo-oxidation.

16. A method according to claim 15 **characterised in that** the dialysis machine is rinsed after disinfecting has been effected with ultra-pure water.

## Revendications

1. Appareil de dialyse avec des moyens pour la génération en ligne d'au moins un agent de désinfection utilisable pour la désinfection de l'appareil de dialyse,
**caractérisé en ce**
**que** les moyens comprennent un générateur (3) pour la production de NaOCl par électrolyse du NaCl et que l'appareil de dialyse présente en outre des moyens (6) pour la décomposition de l'agent de désinfection généré en ligne en produits de décomposition écologiquement acceptables.

2. Appareil de dialyse selon la revendication 1, **caractérisé en ce qu'**il comprend des moyens de production d'ozone, en particulier une électrode en dioxyde de plomb ou un dispositif pour réaliser un procédé Siemens.

3. Appareil de dialyse selon l'une des revendications précédentes, **caractérisé en ce qu'**il comprend un système de photo-oxydation par U.V.

4. Appareil de dialyse selon l'une des revendications précédentes, **caractérisé en ce qu'**il comprend un catalyseur (6) pour la décomposition catalytique des agents de désinfection.

5. Appareil de dialyse selon la revendication 4, **caractérisé en ce qu'**il comprend un catalyseur pour la décomposition catalytique du NaOCl.

6. Appareil de dialyse selon la revendication 5, **caractérisé en ce qu'**il comprend un système de conduites par le biais duquel le NaCl produit par décomposition catalytique peut être de nouveau amené au générateur de NaOCl (3).

7. Appareil de dialyse selon l'une des revendications précédentes, **caractérisé en ce qu'**il comprend un système de conduites, par le biais duquel les produits de décomposition résultant de la décomposition du au moins un agent de désinfection, qui sont réutilisables pour la génération en ligne de l'agent de désinfection, peuvent être réintroduits dans les moyens pour la génération en ligne d'agents de désinfection.

8. Appareil de dialyse selon l'une des revendications précédentes, **caractérisé en ce qu'**il comprend des moyens (5) pour déterminer la teneur en agents de désinfection ou bien la présence d'agents de désinfection dans un liquide de désinfection utilisé pour la désinfection, dans un liquide de rinçage utilisé pour le rinçage du liquide de désinfection et/ou dans un liquide de dialysat utilisé dans le circuit de dialysat.

9. Appareil de dialyse selon la revendication 8, **caractérisé en ce qu'**il comprend une électrode rédox pour déterminer la teneur en agent de désinfection.

10. Procédé de désinfection d'un appareil de dialyse, dans lequel les zones de l'appareil de dialyse à maintenir dépourvues de germes sont désinfectées avec un agent de désinfection, l'agent de désinfection étant produit en ligne par des moyens pour la génération d'au moins un agent de désinfection,
**caractérisé en ce**
**que** la production de l'agent de désinfection comprend la production de NaOCl par électrolyse du NaCl et qu'il se produit une décomposition de l'agent de désinfection généré en ligne en produits de décomposition écologiquement acceptables après le procédé de désinfection dans l'appareil de dialyse.

11. Procédé selon la revendication 10, **caractérisé en ce que** les zones de l'appareil de dialyse à maintenir exemptes de germes sont désinfectées par addition de NaOCl, à titre de liquide de désinfection, et que le NaOCl utilisé à cette occasion est ensuite décomposé catalytiquement en NaCl et en autres produits réactionnels.

12. Procédé selon la revendication 11, **caractérisé en ce que** le NaCl produit par décomposition catalytique peut être au moins partiellement réutilisé pour la production du NaOCl.

13. Procédé selon la revendication 10, **caractérisé en ce que** l'ozone est utilisé à titre d'agent de désinfection.

14. Procédé selon l'une des revendications 10 à 13, **caractérisé en ce que** la présence d'agents de désinfection est constatée.

15. Procédé selon l'une des revendications 10 à 14, **caractérisé en ce que** de l'eau ultrapure est produite par photo-oxydation.

16. Procédé selon la revendication 15, **caractérisé en ce que** l'appareil de dialyse est rincé avec de l'eau ultrapure, une fois la désinfection réalisée.
